# EUROPEAN PATENT APPLICATION

(11) **EP 2 048 144 A1**
(43) Date of publication of application: **15.04.2009**
(21) Application number: 07019823.9
(22) Date of filing: 10.10.2007
(51) Int. Cl.: C07D 401/12

(54) **Process for the preparation of optically pure omeprazole via salt formation with a chiral amine**

(71) Applicant: KRKA, tovarna zdravil, d.d., Novo mesto, 8501 Novo mesto (SI)
(72) Inventor: Veverka, Miroslav, 847 08 Bratislava (SK); Veverková, Eva, 847 08 Bratislava (SK); Kotar-Jordan, Berta, 8311 Kostanjevica na Krki (SK); Janez, Smodis, 8000 Novo mesto (SK); Zupet, Rok, 1211 Ljubljana (SK)
(74) Representative: UEXKÜLL & STOLBERG

(57) **Abstract**

The present invention relates to a process for the preparation of substantially optically pure omeprazole, or a pharmaceutically acceptable salt or solvate thereof. The invention also relates to a process for preparing a pharmaceutical composition comprising the substantially optically pure omeprazole or the pharmaceutically acceptable salt or solvate thereof and to intermediates useful for the preparation of optically pure omeprazole.

## Description

The present invention relates to a process for the preparation of optically pure or optically enriched omeprazole and the pharmaceutically acceptable salts and solvates thereof by optical resolution. The invention also provides intermediates useful for the preparation of optically pure omeprazole.

### Background of the invention

Omeprazole is the common name for 5-methoxy-2-[[4-methoxy-3,5-dimethyl-2-pyridyl)methyl]sulfinyl]-1H-benzimidazole. Omeprazole was first described in EP-A-0 005 129 and is well known as an effective gastric acid secretion inhibitor.

The sulfur atom of omeprazole is chiral. Thus, omeprazole exists in two enantiomeric forms, i.e. the (R)-enantiomer and the (S)-enantiomer, otherwise known as (R)-omeprazole and (S)-omeprazole, respectively. (S)-Omeprazole is also referred to as esomeprazole.

Using non-stereoselective synthesis, omeprazole is obtained as a racemic mixture. Due to differences in pharmacokinetic properties of the (R)- and (S)-isomers of omeprazole, there is a general demand for methods allowing for the preparation of optically pure omeprazole.

Methods for the separation of the enantiomers of omeprazole are known in the art.

DE-A-40 35 455 (BYK GULDEN LOMBERG CHEM. FAB.) relates to a resolution process for obtaining optically pure omeprazole via formation of diastereomeric ethers.

WO-A-94/27988 discloses optical resolution of omeprazole using esters of omeprazole with chiral organic acids such as (R)-mandelic acid.

WO-A-96/02535 discloses a process for the preparation of the single enantiomers of omeprazole by asymmetric oxidation of the corresponding "prochiral" sulfide. The process employs an oxidizing agent in the presence of a chiral titanium complex.

WO-A-97/02261 discloses a process for the optical purification of certain enantiomerically enriched benzimidazole derivatives using a crystallization method.

WO-A-02/098423 relates to an inclusion complex of (S)-omeprazole with cyclodextrin.

CN-A-1223262 relates to a process for the preparation of certain optically pure benzimidazole derivatives by inclusion complexation with binaphthyl phenol derivatives.

WO-A-2007/074099 relates to an analogous process using (S)-1,1,2-triphenyl-1,2-ethanediol.

Cinchona alkaloids have found a number of applications as resolving agents for the fractionated crystallization of chiral acids as diastereomeric salts [P. Newman, Optical Resolution Procedures for Chemical Compounds, Volume 2, Part I and II, Optical Resolution Information Center, Manhattan College, Riverdale, N.Y. 10471, 1981]. For example, the racemic panthotenic acid has been resolved by means of quinine or cinchonidine methohydroxide as described in J. Amer. Chem. Soc. 1941, 63, 1237. Resolution of racemic substituted γ-butyrolactone with cinchona methohydroxide is described in J. Amer. Chem. Soc. 1941, 63, 1368.

IPCOM000126473D discloses an optical resolution process wherein the diastereomeric pair of omeprazole N-benzyl cinchoninium salts is prepared by reactions under non-homogeneous conditions or under conditions employing a large excess of omeprazole. These procedures have not been found practical and economical on an industrial scale.

Thus, there remains a need for an improved process for preparing substantially optically pure omeprazole that does not suffer from the disadvantages of the known methods.

### Description of the invention

It is an object of the present invention to provide a process for preparing substantially optically pure omeprazole which process can advantageously be applied on an industrial scale.

It has surprisingly been found that this object can be achieved by a process for the preparation of substantially optically pure omeprazole, or a pharmaceutically acceptable salt or solvate thereof, comprising the steps of:
(a) contacting omeprazole or a salt thereof with a resolving agent selected from a chiral amine or a chiral quaternary ammonium salt in a solvent to give a diastereomeric pair of omeprazole ammonium salts;
(b) separating the diastereomeric pair of omeprazole ammonium salts to give a substantially optically pure omeprazole ammonium salt;
(c) converting the substantially optically pure omeprazole ammonium salt to obtain substantially optically pure omeprazole or a pharmaceutically acceptable salt or solvate thereof; and
(d) optionally transforming the substantially optically pure omeprazole into a pharmaceutically acceptable salt or solvate thereof.

The process of the present invention offers several advantages. It proceeds with high yield and high enantiomeric purity > 90% e.e. Furthermore, the resolving agents can be readily recovered after optical resolution. The process is easily industrialisable using tailored chiral amines or chiral quaternary ammonium salts and can be carried out under very mild, homogenous conditions. Moreover, the process does not require toxic solvents and can be applied without chromatographic separations.

A "substantially optically pure" isomer in the context of the present invention means an isomer with a diastereomeric excess (d.e.) or enantiomeric excess (e.e.) acceptable for a chiral compound prepared on industrial scale. Such d.e. and e.e. values are readily determined by a person skilled in the art. Usually, a process is suitable for preparation on industrial scale with an d.e. or e.e. of at least 85 %, preferably of at least 90 % and more preferably of at least 95 %.

Omeprazole used in the present invention can be prepared by any known methods, for example such as those disclosed in EP-A-5129, EP-A-103 553, EP-A-302 720, EP-A-369 208, EP-A-533 752, EP-A-533 264 and EP-A-484 265. The crystal form of omeprazole can be A, B or C according to WO-A-99/08500; Ohishi et al., Acta Cryst. 1989, C45, 1921-1923; and WO-A-2002/085889 respectively.

In one embodiment of the invention, the resolving agent is selected from the group consisting of (-)-brucine, (+)-α-methylbenzylamine, (-)-ephedrine, N,N-dimethylephedrine, bis-(1-phenylethyl)amine, cinchona bases, derivatives thereof and quaternary salts thereof.

In a further preferred embodiment, the resolving agent is a quaternary cinchona salt of formula (**I**): 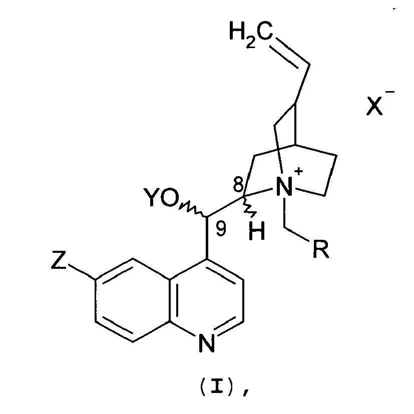 wherein
Z is hydrogen or methoxy;
Y is hydrogen, benzyl or allyl;
R is hydrogen or phenyl;
X is iodine, bromine, chlorine or hydroxy;
and wherein the configuration at C(8) and C(9) is independently (R) or (S).

Quaternary salts of cinchona alkaloids as resolving agents are inexpensive, commercially available or can easily be prepared in both enantiomeric forms, and are also non-toxic and recoverable. It is particularly preferred that the resolving agent is a quaternary salt of a cinchona base selected from the group consisting of (-)-quinine, (+)-quinidine, (+)-cinchonine and (-)-cinchonidine.

According to the process of the invention, racemic or optionally enantiomerically enriched omeprazole or a salt thereof is contacted with a resolving agent as defined above in a suitable solvent. The contacting may be carried out in a conventional manner such as under stirring at ambient temperature.

In a preferred embodiment, the solvent of step (a) comprises at least one solvent which is selected from the group consisting of (C₁-C₅) alcohols, (C₃-C₆) ketones, acetonitrile, dimethylformamide, aromatic (C₆-C₉) hydrocarbons, tetrahydrofuran, aliphatic (C₁-C₄) esters, halogenated aliphatic (C₁-C₉) hydrocarbons and optionally comprises up to 50 vol-% water. More preferably, said solvent is selected from the group consisting of methanol, ethanol, acetone, butanone, dimethylformamide, tetrahydrofuran, dichloromethane, and acetonitrile. In a particularly preferred embodiment, the solvent comprises no more than 35 vol-%, preferably no more than 20 vol-%, most preferably no more than 15 vol-% water.

The resolving agent may generally be applied in any suitable molar amount. In a preferred embodiment, the resolving agent is used in an amount of at least 0.7 equivalents, particularly at least 0.9 equivalents, most preferably at least 1 equivalent, based on the molar amount of omeprazole.

It is possible to recover the diastereomeric pair of omeprazole ammonium salts between steps (a) and (b) and such optional recovery may generally be carried out using conventional measures such as filtration and/or removal of solvent under reduced pressure. In a preferred embodiment, this recovery comprises evaporation of solvent(s) under reduced pressure.

In one embodiment of step (b), separation of the diastereomeric pair of omeprazole ammonium salts comprises crystallizing omeprazole ammonium salt from a suitable solvent for crystallization. Preferably, the solvent for crystallization comprises at least one solvent selected from the group consisting of (C₁-C₅) alcohols, (C₃-C₆) ketones, acetonitrile, dimethylformamide, aromatic (C₆-C₉) hydrocarbons, tetrahydrofuran, aliphatic (C₁-C₄) esters, halogenated aliphatic (C₁-C₉) hydrocarbons. Particularly, the solvent for crystallization of step (b) may be the same or different from the solvent of step (a).

In a further preferred embodiment, step (b) is repeated at least once, such as once, twice, three times, etc. Thus, the omeprazole ammonium salt is recrystallized, thereby resulting in a considerable rise in d.e. reproducibly giving omeprazole ammonium salt of > 90 % d.e. Thus, omeprazole ammonium salt obtained from a first crystallization is typically subjected to recrystallization. Recrystallization can be effected in the same solvent system as defined above and can be carried out at a temperature in the range of from ambient temperature to the reflux temperature of the solvent. Finally the batch is usually filtered and washed with solvent.

Crystallization and recrystallization of omeprazole ammonium salt from a suitable solvent system may be carried out in a conventional manner. Diastereomeric salt resolution proved to be a dynamic process with the diastereomeric excess (d.e.) of the crystals enriching over time. Typically, a sample of the solids is isolated from the crystallization mixture for d.e. determination (e.g. d.e. criterion >90% d.e.).

In a preferred embodiment of step (c), converting the omeprazole ammonium salt comprises contacting the omeprazole ammonium salt with at least one compound selected from the group consisting of an acid, e.g. hydrochloric acid, an acidic salt, e.g. MgCl₂ or NH₄Cl, and an acidic ion exchanger, e.g. Dowex 50W H⁺.

Preferably, the conversion of step (c) is carried out in a suitable solvent system comprising water and at least one water-immiscible solvent. More preferably, the water-immiscible solvent is selected from the group consisting of aromatic (C₆-C₉) hydrocarbons, aliphatic (C₂-C₈) ethers, aliphatic (C₁-C₄) esters, halogenated aromatic or aliphatic (C₁-C₉) hydrocarbons, and mixtures thereof. Particularly preferred are toluene, dichloromethane, ethyl or butyl acetate and tert-butyl methyl ether. When the conversion step is carried out in the presence of a water-immiscible solvent, further steps of washing and removal of the solvent may be required.

Enantiomerically enriched esomeprazole can be further optically purified by preparative chromatography, such as HPLC or SMB chromatography. For example, methods generally disclosed in WO-A-2003/051867, CN-A-1683368 or WO-A-2007/071753 can be used.

Alternatively, without prior neutralization an alkaline or alkaline earth metal salt of omeprazole can be isolated directly in a substantially optically pure form from the aqueous phase by conventional methods. The alkaline metal cation can be replaced by another metal cation prior to isolation by treatment with an appropriate alkaline earth metal salt such as an alkaline earth halide such as magnesium chloride as described for example in WO-A-94/27988.

Furthermore, neutral optically pure omeprazole can optionally be subsequently transformed into a pharmaceutically acceptable optically pure omeprazole salt with an appropriate base by conventional methods, in particular into an alkaline, alkaline earth or transitional metal salt such as a lithium, sodium, potassium, magnesium, calcium, barium or zinc salt. Sodium and magnesium salts are particularly preferred. Other non-metal salts such as amine salts can also be prepared. Above mentioned pharmaceutically acceptable salts and methods for there preparation are generally disclosed in WO-A-94/27988, EP-A-984 957, EP-A-1 375 497, WO-A-2004/020436, WO-A-2004/037253, WO-A-2004/046134, WO-A-2004/089935, EP-A-1 273 581, EP-A-1 149 090, WO-A-2004/111029, WO-A-2006/001753, WO-A-2006/1755, WO-A-2004/099182, CN-A-1683369, WO-A-2006/073779, WO-A-2004/099181, WO-A-2003/074514, WO-A-2005/023796, WO-A-2005/23797, DE-A-10 2005 008412, EP-A-1 726 305 and RD462058.

In a particularly preferred embodiment of the present invention, the pharmaceutically acceptable solvate is (S)-omeprazole magnesium dihydrate form A. Starting from (S)-omeprazole magnesium prepared according to the process described above, (S)-omeprazole magnesium dihydrate form A can be obtained according to the method disclosed in example 6 of WO-A-98/54171.

According to the process of the present invention, selective formation of either one of the two diastereomeric ammonium salts can be advantageously controlled by using a suitable chiral quaternary ammonium salt. By way of example, using a cinchoninium salt as resolving agent will result in crystallization of the (S)-compound whereas using a cinchonidinium salt as resolving agent will result in crystallization of the (R)-compound. Thus, the process of the present invention can be controlled such that crystals of the (S)-compound are obtained from fractionated crystallization, and crystals of the (R)-compounds may subsequently be isolated from the mother liquor. Alternatively, the process can be controlled such that crystals of the (R)-compound are obtained from fractionated crystallization and crystals of the (S)-compound may subsequently be isolated from the mother liquor.

According to one particularly preferred embodiment of the process of the invention, racemic compound is dissolved in ethanol, acetone, dimethylformamide, dichloromethane, tetrahydrofuran or the like organic solvent. The reaction is not sensitive to water, thus a limited amount of water may be there such as up to 50 vol-%. N-benzylcinchonine hydroxide solution in an aforementioned solvent is added thereto in an equivalent molar amount, and then the mixture is concentrated in vacuo to obtain N-benzyl cinchoninium salt of the corresponding compound as a solid material. The isolated substance is dissolved in ethanol, isopropanol, butanol, methyl ethyl ketone, ethyl acetate or mixtures thereof, and the solution is left to stand to form crystals. The crystals are recovered by filtration of the solution and subjected to recrystallization to obtain the N-benzylcinchoninium salt of (S)-compound. The salt is treated with hydrochloric acid and recrystallized from an organic solvent in a conventional manner to obtain the desired (S)-compound. After the (S)-compound is filtered off, the mother liquor may be concentrated by evaporation of solvent to obtain N-benzyl cinchoninium salt of the compound mainly containing the (R)-compound and which may subsequently be treated with hydrochloric acid to obtain crystals of the compounds containing mainly the (R)-compound.

According to another particularly preferred embodiment of the process of the invention, an alkali metal salt of the racemic starting compound is dissolved in methanol, dimethylformamide, acetonitrile, tetrahydrofuran or the like organic solvent and/or water, and N-methyl cinchonidine iodide in an aforementioned organic solvent is added thereto in an equivalent molar amount. Water-immiscible solvents also may be occasionally used. The organic solvent is then concentrated in vacuo to obtain the N-methyl cinchonidine salt of the corresponding compound as a solid material. The diastereomeric pair of salts is dissolved in ethanol, isopropanol, butanol, methyl ethyl ketone, ethyl acetate or the like organic solvent and left to stand to obtain crystals which are recrystallized to obtain N-methylcinchonidinium salt of the (R)-compound. After the (R)-compound is filtered off, the mother liquor may be concentrated by evaporation of solvent to obtain N-methylcinchonidinium salts of the compounds mainly containing the (S)-compound. The (S)-salt is acidified with hydrochloric acid and recrystallized from an organic solvent to obtain the desired (S)-compound. Alternatively the desired (S)-compound is liberated straightforward from the mother liquor by addition of acid.

The present invention also relates to a process for preparing a pharmaceutical composition comprising substantially optically pure omeprazole, or a pharmaceutically acceptable salt or solvate thereof, in combination with at least one pharmaceutically acceptable carrier, which includes the step of preparing the substantially optically pure omeprazole or the pharmaceutically acceptable salt or solvate thereof according to the process of the invention. Preferably, the solvate is (S)-omeprazole magnesium dihydrate form A.

The particle size of the (S)-omeprazole and pharmaceutically acceptable salts or solvates used in the processes of the present invention is preferably in the range of 0.1-250 microns. If smaller particles are needed, they can be milled or micronized optionally together with other excipients. During this process the mechanical force on the particle surface leads to a particle size reduction. For this purpose an air jet mill, a ball mill or a hammer mill are commonly used as milling equipment. The basic principle of the treatment in an air jet mill is collision and attrition between particles suspended within a high velocity air stream, which introduces the power to the milling chamber.

The pharmaceutical compositions according to the invention are preferably in the form of pellets or tablets, in particular those which have an enteric coating layer. The pellets can be filled into hard gelatine capsules or sachets which hence also represent preferred embodiments of the composition. The tablets can be prepared from the pellets or from a powder mixture.

The pellets preferably comprise
(a) a core including the pharmaceutically acceptable salt or solvate according to the invention,
(b) optionally at least one separating layer,
(c) at least one enteric layer, and
(d) optionally at least one over-coating layer.

The tablets preferably comprise
(a) a core including the pharmaceutically acceptable salt or solvate according to the invention,
(b) optionally at least one separating layer, and
(c) at least one enteric layer.

In a preferred embodiment of both, the pellets and the tablets, the pharmaceutically acceptable salt is included in the core in form of a layer covering the core.

In a further embodiment, the pharmaceutical compositions are in the form of multiple-unit tablets which are obtainable by compressing a mixture of the pellets with tablet excipients.

The present invention also relates to intermediates useful for the preparation of optically pure omeprazole.

According one embodiment the intermediate is an omeprazole ammonium salt, wherein the ammonium moiety is derived from a chiral amine selected from the group consisting of (-)-brucine, (+)-α-methylbenzylamine, (-)-ephedrine, N,N-dimethylephedrine and bis-(1-phenylethyl)amine.

According to another embodiment, the intermediate is an omeprazole ammonium salt having the formula (**II**): 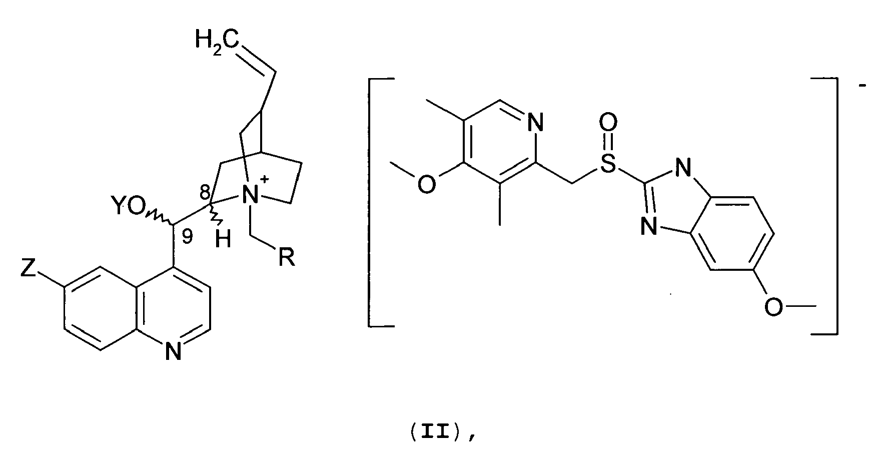 wherein:
the configuration at C(8), C(9) and the sulfur atom is independently (R) or (S);
   Z is hydrogen or methoxy;
   Y is benzyl or allyl; and
   R is hydrogen or phenyl.

According to another embodiment, the intermediate is an omeprazole ammonium salt having the formula (**III**): 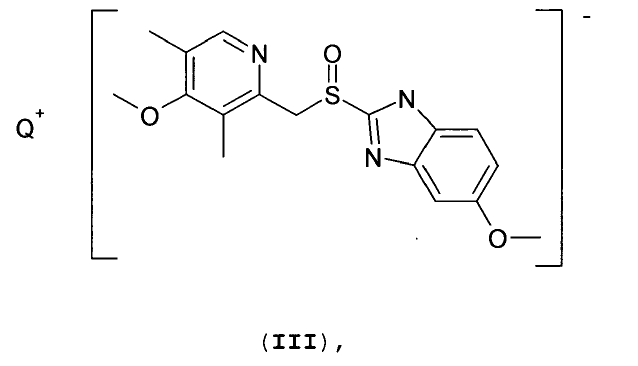 wherein the configuration at the sulfur atom is independently (R) or (S) and wherein Q⁺ is selected from the group consisting of:

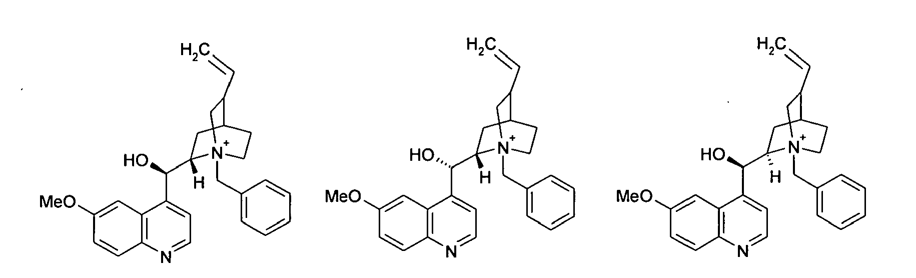

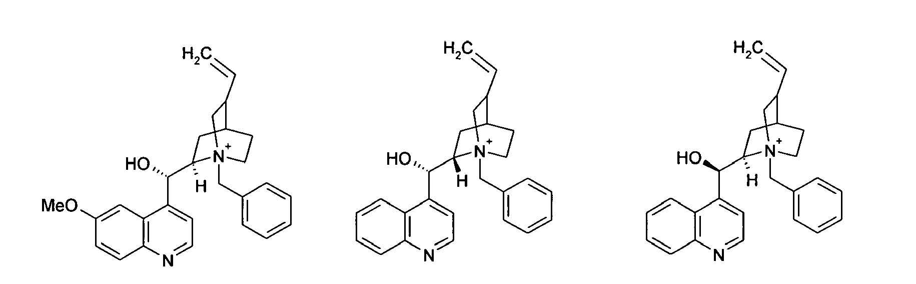

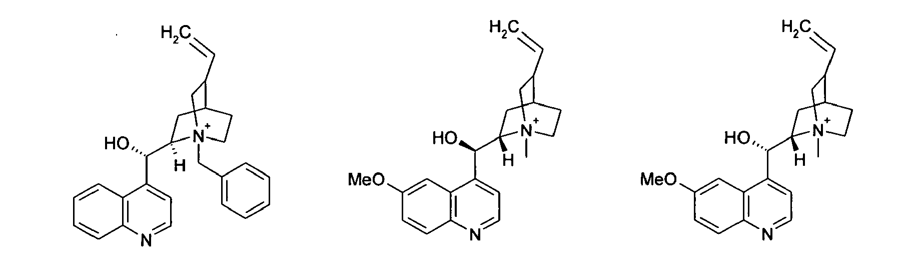

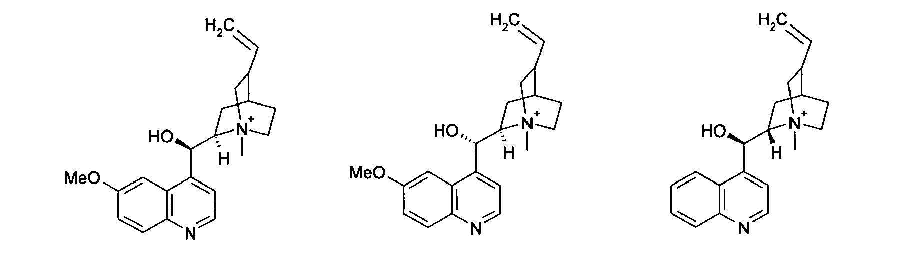

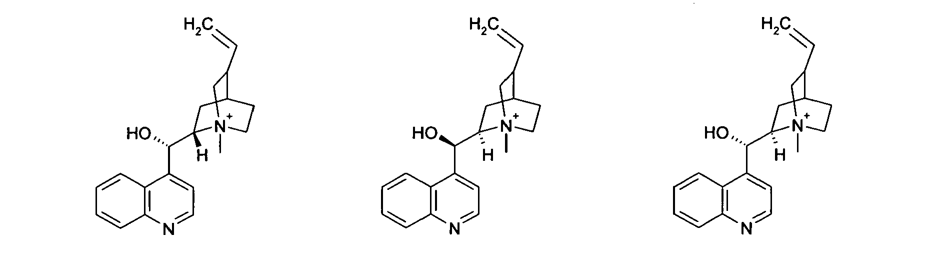

The present invention will further be illustrated by means of the following examples.

### Examples

### Example 1

Optical resolution of racemic omeprazole

To a solution of omeprazole (5.92 g) in 200 ml of methanol, a methanolic solution of N-benzylcinchoninium hydroxide (7.2 g in 100 ml MeOH) was added. After stirring the mixture at room temperature for 0.5 hours, the solution was evaporated in vacuo to dryness at a temperature of 45°C. The resulting pale yellow solid of diastereomeric pair salt (13.2 g, m.p. 183-186°C) was twice recrystallized from n-butanol/2-propanol (1:7) to give 5.2 g of (S)-omeprazole N-benzylcinchoninium salt (m.p. 205-207°C, 99% d.e. determined by chiral HPLC). A second crop (0.7 g, m.p. 202-204°C, 98.5% d.e. determined by chiral HPLC) was obtained by concentrating the combined mother liquor and washings, and twice allowing recrystallization to proceed at room temperature for 24 hours.
The obtained salt (5.2 g) was dissolved in a mixture of 25 ml of ethanol and 2.5 ml of water. To the solution was added 0.3 N hydrochloric acid solution under stirring in an ice bath to a pH value of 3-3.5, and the mixture was stirred at a temperature of 0°C for 1 hour. The solvent was evaporated in vacuo to give a semisolid mass, to which 20 ml of ethyl acetate were added. After stirring the mixture at room temperature, the solid was filtered off. N-Benzylcinchoninium chloride was thus recovered in a yield of 99 % having a purity of 99 % (determined by HPLC).
The organic layer was separated and evaporated, and the obtained solidified mass was recrystallized from toluenecyclohexane (1:3) to give 2.1 g of (S)-omeprazole (m.p. 65-80°C, 99 % e.e. (determined by chiral HPLC), [α] -41.5° (0.17, methanol)).

### Example 2

Optical resolution of racemic omeprazole

A solution of N-benzylcinchoninium chloride (5.9 g) in ethanol/water (9:1, 100 ml) was passed through an Ambersep 900 OH (11 g) column. To the obtained solution omeprazole (4.6 g) was added. After stirring the mixture at room temperature for 1 hour, the mixture was evaporated in vacuo to dryness at a temperature of 45°C. To the obtained solid mass was then added toluene (20 ml), and the resulting mixture was evaporated to dryness. The resulting pale yellow solid (9.7 g) was dissolved in 114 ml of n-butanol/2-propanol (1:7).
After stirring the mixture at room temperature overnight, crystals deposited out therein were obtained through a filtration to give 4.7 g of enriched (S)-omeprazole N-benzylcinchoninium salt.
The material was twice recrystallized from n-butanol/2-propanol (1:7) to give 3.3 g of (S)-omeprazole N-benzylcinchoninium salt (m.p. 203-205°C, 98.5% d.e. (determined by chiral HPLC), [α] +52° (1.0, methanol)).
By concentrating the mother liquor and washings and allowing recrystallization to proceed at room temperature for 24 hours, 0.6 g of (S)-omeprazole N-benzylcinchoninium salt was recoverd.
The obtained salt (3.2 g) was dissolved in 25 ml of ethanol. To the solution was added Dowex 50W, H⁺ form under stirring in an ice bath to a pH value of 4, and the mixture was stirred at a temperature of 0°C for 0.5 hours. The reaction mixture was filtered, washed with 2 x 5 ml of ethanol, and the filtrate was evaporated in vacuo to give a semisolid mass. Solidified mass was recrystallized from toluene/cyclohexane (1:3) to give 0.85 g of (S)-omeprazole (99.5% e.e. (determined by chiral HPLC)).

### Example 3

### Optical resolution of racemic omeprazole

To a mixture of omeprazole (0.79 g) and sodium hydroxide (0.11 g) in methanol (35 ml) a solution of N-methylcinchonidinium iodide (1.0 g) in methanol (30 ml) was added.
After stirring the mixture at room temperature for 1 hour, the mixture was cooled down in a refrigerator, filtered (Celite) and then evaporated in vacuo to dryness. The resulting pale yellow solid (m.p. 106-110°C, [α] -65° (1.0, methanol)) was dissolved in 82 ml of acetone/butyl acetate (7:1).
After stirring the mixture at room temperature overnight, crystals deposited out therein were obtained through a filtration to give 0.7 g, of enriched (R)-omeprazole N-methylcinchonidinium salt.
The material was recrystallized from acetone/butyl acetate (7:1) to give 0.5 g of enriched (R)-omeprazole N-methylcinchonidinium salt (m.p. 193-196°C, [α] -47° (1.0, methanol)).
(S)-omeprazole N-methylcinchonidinium salt was obtained from the concentrated mother liquor, i.e. the filtrate after filtration of the crystalline mass.
The crystalline (S)-omeprazole N-methylcinchoninium salt was twice recrystallized from 2-butanone/butylacetate (1:3) and a small volume of ethanol; m.p 95-107°C, 98.5% d.e. (determined by chiral HPLC).
Liberation of (S)-omeprazole was accomplished analogously to example 2.

### Example 4

### Optical resolution of racemic omeprazole

Analogously to example 2, a dimethylformamide solution of N-methylcinchoninium iodide was used.
After stirring the reaction mixture at room temperature for 1 hour, the mixture was evaporated in vacuo to dryness and then washed with diethyl ether (2 x 20 ml) to obtain a pale yellow solid (m.p. 118-129°C, [α] +104° (1.0, methanol)).
The resulting salt was twice recrystallized from 2-butanone and a small volume of ethanol to obtain crystalline (S)-omeprazole N-methylcinchoninium salt (m.p. 110-125°C, 99% d.e. (determined by chiral HPLC), [α] +21° (0.7, methanol)). To a suspension of the obtained salt (2.5 g) in toluene (70 ml) was added 3% aqueous hydrochloric acid at a temperature of 5-10°C to a pH value of 3. The mixture was stirred, filtered and the organic phase was decanted. The organic phase was extracted with 10 % sodium hydroxide solution (2 x 10 ml) and after cooling to 5°C acidified with glacial acetic acid with vigorous stirring. Stirring was continued for 0.5 h, then the solution was seeded with (S)-omeprazole (100 mg) at 5°C and filtered. The collected solid was washed with water (2 x 5 ml) and dried in vacuo at ambient temperature to afford (S)-omeprazole (0.95 g, 99% e.e.) as a slightly brown solid.

### Example 5

### Optical resolution of racemic omeprazole

Analogously to the example 1, a tetrahydrofuran/water (4:1) solution of N-benzylquinidine hydroxide was used.
After stirring the reaction mixture at room temperature for 1 hour, the mixture was evaporated in vacuo at a temperature of 45°C. The resulting pale yellow solid was washed with diethyl ether (210 ml) and then filtered (m.p. 80-105°C, [α] +93° (1.0, methanol)).
The crystalline (S)-omeprazole N-benzylquinidinium salt crystallized slowly from 2-butanone/diisopropylether (1:3) and a small volume of ethanol; m.p. 110-125°C, 98% d.e. (determined by chiral HPLC).

### Example 6

### Preparation of (R,S)-omeprazole N-benzylquinine salt

To a solution of omeprazole (2.1 g) in dichloromethane (150 ml) a solution of sodium hydroxide (0.26 g) in water (7.5 ml) was added. To the resulting clear two phase system was added N-benzylquininium bromide (3 g), and the reaction mixture was stirred for 0.5 hours. The organic phase was separated and evaporated in vacuo to dryness at a temperature of 45°C. The obtained pale yellow solid was filtered and washed on a Büchner funnel with hot toluene (2 x 10 ml) to give the diastereomeric pair of omeprazole N-benzylquinine salt (4.7 g, m.p. 106-118°C).

### Example 7

Preparation of (R,S)-omeprazole N,N-dimethylephedrinium salt

Analogously to example 6, N,N-dimethylephedrinium bromide was used.
The diastereomeric pair of omeprazole N,N-dimethylephedrinium salt (m.p. 104-116°C, [α] -13° (1.0, methanol)) was obtained as a pale yellow solid.

### Example 8

### Preparation of (R,S)-omeprazole O-allyl-N-benzylcinchonidinium salt

Analogously to example 3, O-allyl-N-benzylcinchonidinium bromide was used.
The diastereomeric pair of omeprazole O-allyl-N-benzylcinchonidinium salt (m.p. 85-106°C) was obtained as a pale yellow solid.

### Example 9

### Preparation of (R,S)-omeprazole N-methylquininium salt

Analogously to example 1, an aqueous quinine methohydroxide solution prepared according J. Am. Chem. Soc. 1941, 63, 1368 was added to the methanol solution of (R,S)-omeprazole.

### Example 10

Further purification of enantiomerically enriched omeprazole by preparative HPLC

| Batch | 1 | 2 | 3 | 4 |
|---|---|---|---|---|
| Equipment | Preparative Knauer HPLC System | | | |
| Data evaluation | Eurochrome | | | |
| Feed (S:R) | 98:2 | 95:5 | 80:20 | 50:50 |
| Column | "Amy Coat", Kromasil; 10 µm; 150X4,6 mm | | | |
| Mobile phase | Methanol + 0.1% Diethylamine (DEA) | | | |
| Sample preparation | concentration 30mg/ml esomeprazole in Methanol + 0,1% DEA | | | |
| Sample loading (mg/run) | 24 | 32 | 24 | 6 |
| Post run | 0 min | | | |
| Column temperature | 25°C | | | |
| Flow rate | 1.5 mL/min | | | |
| Detection | 254 nm; 300 nm | | | |
| Injection volume | from 0.1 to 4.5ml per run | | | |
| Yield, purity (S) | 13.18 | 22.90 | 11.72 | 1.4 |
| (mg, % S) | 100 | 99.55 | 99.73 | 99.92 |

### Example 11

### Preparation of amorphous (S)-omeprazole magnesium

### Variant (a)

50.0 g (0.1447 moles) (S)-omeprazole was dissolved in 500 ml water with 18 ml diethylamine. A solution of 31.47 g (0.0759 moles) magnesium gluconate in 200 ml water was gradually added, and the mixture was stirred for 3 hours at 20-25°C. The product was filtered and washed with 100 ml of water. The wet product was macerated in 500 ml water, filtered and dried to obtain 40.0 g of (S)-omeprazole magnesium as amorphous form.

### Variant (b)

50 g (0.1447 moles) (S)-omeprazole was dissolved in 500 ml of water with 18 ml diethylamine. A solution of 13.32 g (0.0723 moles) magnesium bromide in 200 ml of water was gradually added, and the mixture was stirred for 12 hours at 20-25°C. The product was filtered and washed with 100 ml of water. The wet product was macerated in 500 ml water, filtered and dried to obtain 45.0 g of (S)-omeprazole magnesium as amorphous form.

### Variant (c)

5.0 g (0.01447 moles) (S)-omeprazole was dissolved in a solution of 50 ml water and 2.52 g (0.01447 moles) arginine. A solution of 1.33 g (0.0072 moles) magnesium bromide in 20 ml water was gradually added, and the mixture was stirred for 12 hours at 20-25°C. The product was filtered and washed with 10 ml of water. The wet product was macerated in 50 ml water, filtered and dried to obtain 4.6 g of (S)-omeprazole magnesium as amorphous form.

### Example 12

Preparation of (S)-omeprazole magnesium trihydrate.

Magnesium (0.10 g) was activated with iodine and reacted with absolute methanol (10 ml) to give a solution. Reaction mixture was diluted with methanol (15 ml) and added dropwise to a solution of (S)-omeprazole (2.6 g) in methanol (80 ml) for 30 min at 10°C. To the reaction mixture methanol was added (75% water solution, 15 ml) and then filtered clear (Celite) and concentrated by evaporation under reduced pressure. Evaporation gave a white solid of the (S)-omeprazole magnesium trihydrate (m.p. 198-201°C).

### Example 13

### Particle size of (S)-omeprazole magnesium dihydrate form A

Starting from (S)-omeprazole obtained according to the process of the present invention, (S)-omeprazole magnesium dihydrate form A was prepared according to example 6 of WO-A-98/54171.

In one variant, the (S)-omeprazole magnesium dihydrate form A was dried without subsequent milling. According to another variant, the (S)-omeprazole magnesium dihydrate form A was milled on Fitz D6A at 2000 rpm. According to a third variant, the (S)-omeprazole magnesium dihydrate form A was dried using a fluid bed apparatus.

The mean particle size, particle size distribution and specific surface area obtained according to the three variants are given in the following table:

| | **unmilled** | **milled** | **fluid bed** |
|---|---|---|---|
| **mean particle size** | **153** µ**m** | **30 µm** | **9** µ**m** |
| d₁₀ | 12,6 µm | 8,3 µm | 3,1 µm |
| d₅₀ | 80,5 µm | 24,3 µm | 7,2 µm |
| d₉₀ | 403,8 µm | 60,2 µm | 15,3 µm |
| **Specific surface area** | **6,1 m²/g** | **7,7 m²/g** | 8,0 **m²/g** |

The dₓ value indicates that a certain percentage X by volume of the particles has a size below a certain limit. For example, a d₉₀ value of 150 µm means that 90 % by volume of the particles have a diameter below 150 µm.

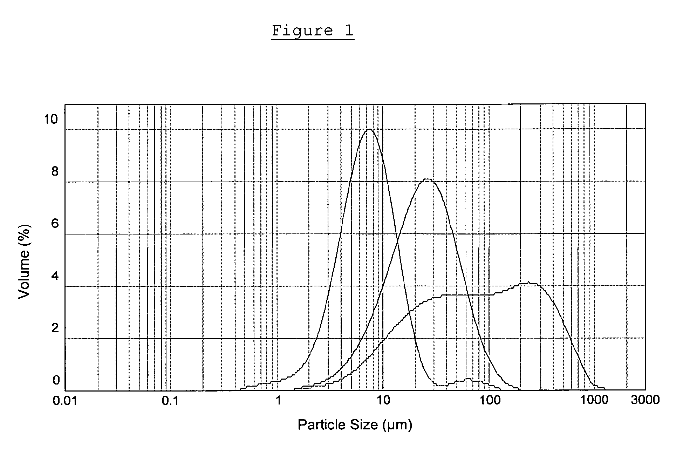

Figure 1 depicts particle size distribution diagrams of unmilled and milled (S)-omeprazole magnesium dihydrate form A.

### PHARMACEUTICAL FORMULATIONS WITH (S)-OMEPRAZOLE MAGNESIUM DIHYDRATE FORM A

### Example 14

| **ingredients** | **composition [g]** | **percentage** |
|---|---|---|
| **Core material** | | |
| (S)-omeprazole magnesium dihydrate form A | 41.29^{*} | 31.95 % |
| Neutral spheres | 71.16 | 55.06% |
| Povidone | 15.00 | 11.61 % |
| Sodium lauryl sulfate | 1.80 | 1.39 % |

| **Separating layer 1** | | |
|---|---|---|
| Core material | 129.25 | 82.90 % |
| Opadry II White (PVA, talc, TiO₂, PEG) (I) | 18.75 | 12.03 % |
| Magnesium hydroxide carbonate heavy (I) | 7.92 | 5.08 % |
| | | |
| **Separating layer 2** | | |
| Core material | 155.92 | 89.22 % |
| Opadry II White (PVA, talc, TiO₂, PEG) (II) | 18.75 | 10.73 % |
| Magnesium hydroxide carbonate heavy (II) | 0.08 | 0.05 % |
| | | |
| **Enteric coating** | | |
| Pellets with separating layer | 174.75 | 69.48 % |
| Methacrylic acid ethyl acrylate copolymer | 62.91 | 25.01 % |
| Glycerol monostearate | 3.15 | 1.25 % |
| Triethyl citrate | 9.44 | 3.75 % |
| Polysorbate 80 | 1.26 | 0.50 % |

| | | |
|---|---|---|
| ^{*}mass of (S) - omeprazole magnesium excluding water | | |

The drug suspension was prepared by suspending (S)-omeprazole magnesium dihydrate form A, which had been obtained according to the invention, into an aqueous solution of povidone and sodium lauryl sulfate. Suspension layering of neutral pellets (e.g. sugar or microcrystalline spheres) was performed in a fluid bed processor using a bottom spray technique (Wurster column) at a batch size of 3.0 kg. The spray operation was stopped when the specified amount of bulk liquid had been sprayed.

The prepared core material was dried until the loss on drying of the pellets was about 1-1.5 %. The pellets were then covered with two separating layers in a Wurster column. The suspensions used for preparing the separating layers both consisted of purified water, magnesium hydroxide carbonate heavy and Opadry powder mixture, the latter being composed of polyvinyl alcohol, talc, titanium dioxide and polyethylene glycol. The coated pellets were dried until the loss on drying was about 1-1.5 %.
An enteric coating suspension was prepared using methacrylic acid ethyl acrylate copolymer (Eudragit L 30 D-55), triethyl citrate, glyceryl monostearate and Polysorbate 80. The suspension was sprayed onto the pellets in a fluid bed apparatus. The spray operation was stopped when the specified amount of bulk liquid had been sprayed, and then drying was carried out in the Wurster column.
The obtained pellets were filled into hard gelatine capsules.

### Example 15

| **ingredients** | **composition [g]** | **composition [%]** |
|---|---|---|
| **Core material** | | |
| (S)-omeprazole magnesium dihydrate form A | 41.29* | 31.94 % |
| Neutral (sugar based) spheres | 71.15 | 55.04 % |
| Povidone | 15.03 | 11.63 % |
| Sodium lauryl sulfate | 1.80 | 1.39 |

| **Separating layer** | | |
|---|---|---|
| Core material | 129.27 | 89.92 % |
| Methylcellulose | 8.00 | 5.56 % |
| Magnesium hydroxide carbonate heavy | 6.50 | 4.52 % |
| | | |

| **Enteric coating** | | |
|---|---|---|
| Pellets with separating layer | 143.77 | 73.21 % |
| Methacrylic acid ethyl acrylate copolymer | 43.13 | 21.96 % |
| Glycerol monostearate | 2.16 | 1.10 % |
| Triethyl citrate | 6.47 | 3.29 % |
| Polysorbate 80 | 0.86 | 0.44 % |

| | | |
|---|---|---|
| ^{*}mass of (S) - omeprazole magnesium excluding water | | |

The drug suspension was prepared by suspending (S)-omeprazole magnesium dihydrate form A, which had been obtained according to the invention, into an aqueous solution of povidone and sodium lauryl sulfate. Suspension layering of sugar spheres was performed in a fluid bed processor using a bottom spray technique (Wurster column) at a batch size of 3.0 kg. The spray operation was stopped when the specified amount of bulk liquid had been sprayed.
The prepared core material was dried until the loss on drying of the pellets was about 1-1.5 %. The pellets were then covered with a separating layer in a Wurster column. The suspension used for forming the separating layer consisted of purified water, methylcellulose and magnesium hydroxide carbonate heavy.
The enteric coating suspension was prepared using methacrylic acid ethyl acrylate copolymer (Eudragit L 30 D-55), triethyl citrate, glyceryl monostearate and Polysorbate 80. The suspension was sprayed onto the pellets in a fluid bed apparatus. The spray operation was stopped when the specified amount of bulk liquid had been sprayed, and then drying was carried out in the Wurster column.
The obtained pellets were filled into HPMC capsules.

### Example 16

| **ingredients** | **composition [g]** | **composition [%]** |
|---|---|---|
| **Core material** | | |
| (S)-omeprazole magnesium dihydrate form A | 41.29^{*} | 36.87 % |
| Cellactose | 56.56 | 50.50 % |
| Microcrystalline cellulose | 8.47 | 7.56 % |
| Croscarmellose sodium | 3.43 | 3.06 % |
| Magnesium stearate | 2.25 | 2.01 % |
| | | |

| **Separating layer** | | |
|---|---|---|
| Core material | 112.00 | 88.53 % |
| Methylcellulose | 11.10 | 8.77 % |
| Titanium dioxide | 0.39 | 0.31 % |
| Iron Oxide Yellow | 0.07 | 0.06 % |
| Propyleneglycol | 2.95 | 2.33 % |
| | | |

| **Enteric coating** | | |
|---|---|---|
| Tablets with separating layer | 126.51 | 92.67 % |
| Methacrylic acid ethyl acrylate copolymer | 6.78 | 4.97 % |
| Macrogol | 0.68 | 0.50 % |
| Talc | 2.54 | 1.86 % |

| | | |
|---|---|---|
| ^{*}mass of (S) - omeprazole magnesium excluding water | | |

The direct compression technology was used to produce tablet cores. The cores were then coated with separating and enteric coating.

### Example 17

| **ingredients** | **composition [g]** | **composition [%]** |
|---|---|---|
| **Core material** | | |
| (S)-omeprazole magnesium dihydrate form A | 41.29^{*} | 31.33 % |
| Neutral (sugar based) spheres | 71.15 | 54.00 % |
| Povidone | 15.03 | 11.41 % |
| Sodium lauryl sulfate | 1.80 | 1.37 % |
| Magnesium hydroxide carbonate heavy | 2.50 | 1.90 % |
| | | |

| **Separating layer** | | |
|---|---|---|
| Core material | 131.77 | 85.42 % |
| Methylcellulose | 8.00 | 5.19 % |
| Magnesium hydroxide carbonate heavy | 6.50 | 4.21 % |
| Povidone | 3.00 | 1.94 % |
| Talc | 5.00 | 3.24 % |

| **Enteric coating** | | |
|---|---|---|
| Pellets with separating layer | 154.27 | 73.21 % |
| Methacrylic acid ethyl acrylate copolymer | 46.28 | 21.96 % |
| Glycerol monostearate | 2.31 | 1.10 % |
| Triethyl citrate | 6.94 | 3.29 % |
| Polysorbate 80 | 0.92 | 0.44 % |

| | | |
|---|---|---|
| ^{*}mass of (S) - omeprazole magnesium excluding water | | |

The drug suspension was prepared by suspending (S)-omeprazole magnesium dihydrate form A, which had been obtained according to the invention, into an aqueous solution of povidone and sodium lauryl sulfate. Suspension layering of sugar spheres was performed in a fluid bed processor using a bottom spray technique (Wurster column) at a batch size of 3.0 kg. The spray operation was stopped when the specified amount of bulk liquid had been sprayed.
The prepared core material was dried until the loss on drying of the pellets was about 1-1.5 %. The pellets were then covered with a separating layer in a Wurster column. The suspension used for preparing the separating layer consisted of purified water, methylcellulose and magnesium hydroxide carbonate heavy.
The enteric coating suspension was prepared using methacrylic acid ethyl acrylate copolymer (Eudragit L 30 D-55), triethyl citrate, glyceryl monostearate and Polysorbate 80. The suspension was sprayed onto the pellets in a fluid bed apparatus. The spray operation was stopped when the specified amount of bulk liquid had been sprayed, and then drying was carried out in the Wurster column.

## Claims

1. A process for the preparation of substantially optically pure omeprazole, or a pharmaceutically acceptable salt or solvate thereof, comprising the steps of:
(a) contacting omeprazole or a salt thereof with a resolving agent selected from a chiral amine or a chiral quaternary ammonium salt in a solvent to give a diastereomeric pair of omeprazole ammonium salts;
(b) separating the diastereomeric pair of omeprazole ammonium salts to give a substantially optically pure omeprazole ammonium salt;
(c) converting the omeprazole ammonium salt to obtain substantially optically pure omeprazole or a pharmaceutically acceptable salt or solvate thereof; and
(d) optionally transforming the substantially optically pure omeprazole into a pharmaceutically acceptable salt or solvate thereof.

2. The process according to claim 1, wherein the resolving agent is selected from the group consisting of (-)-brucine, (+)-α-methylbenzylamine, (-)-ephedrine, N,N-dimethylephedrine, bis-(1-phenylethyl)amine, cinchona bases, and quaternary salts thereof.

3. The process according to claim 1, wherein the resolving agent is a quaternary cinchona salt of formula (I): 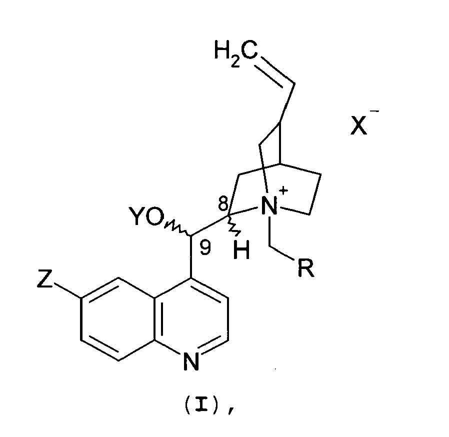 wherein
Z is hydrogen or methoxy;
Y is hydrogen, benzyl or allyl;
R is hydrogen or phenyl;
X is iodine, bromine, chlorine or hydroxy;
and wherein the configuration at C(8) and C(9) is independently (R) or (S).

4. The process according to claim 3, wherein the resolving agent is a quaternary salt of a cinchona base selected from the group consisting of (-)-quinine, (+)-quinidine, (+)-cinchonine and (-)-cinchonidine.

5. The process according to any one of claims 1 to 4, wherein the solvent of step (a) comprises at least one solvent which is selected from the group consisting of (C₁-C₅) alcohols, (C₃-C₆) ketones, acetonitrile, dimethylformamide, aromatic (C₆-C₉) hydrocarbons, tetrahydrofuran, aliphatic (C₁-C₄) esters, halogenated aliphatic (C₁-C₉) hydrocarbons and optionally comprises up to 50 vol-% water.

6. The process according to claim 5, wherein the solvent of step (a) comprises no more than 35 vol-%, preferably no more than 20 vol-%, most preferably no more than 15 vol-% water.

7. The process according to any one of claims 1 to 6, wherein the resolving agent is used in an amount of at least 0.7 equivalents, preferably at least 0.9 equivalents, most preferably at least 1 equivalent, based on the molar amount of omeprazole.

8. The process according to any of claims 1 to 7, wherein between steps (a) and (b) the diastereomeric pair of omeprazole ammonium salts is recovered preferably by evaporation of solvent(s) under reduced pressure.

9. The process according to any of claims 1 to 8, wherein step (b) comprises crystallizing omeprazole ammonium salt from a solvent for crystallization.

10. The process according to claim 9 wherein the solvent for crystallization comprises at least one solvent selected from the group consisting of (C₁-C₅) alcohols, (C₃-C₆) ketones, acetonitrile, dimethylformamide, aromatic (C₆-C₉) hydrocarbons, tetrahydrofuran, aliphatic (C₁-C₄) esters, halogenated aliphatic (C₁-C₉) hydrocarbons.

11. The process according to any of claims 1 to 10, wherein step (c) comprises contacting the omeprazole ammonium salt with at least one compound selected from the group consisting of an acid, an acidic salt and an acidic ion exchanger.

12. The process according to any of claims 1 to 11 wherein the conversion of step (c) is carried out in a solvent system comprising water and at least one water-immiscible solvent.

13. The process according to claim 12, wherein the water-immiscible solvent is selected from the group consisting of (C₆-C₉) aromatic hydrocarbons, aliphatic (C₂-C₈) ethers, aliphatic (C₁-C₄) esters, halogenated aromatic and aliphatic (C₁-C₉) hydrocarbons.

14. The process according to any one of claims 1 to 13 wherein the pharmaceutically acceptable solvate is (S)-omeprazole magnesium dihydrate form A.

15. Process for preparing a pharmaceutical composition comprising substantially optically pure omeprazole, or a pharmaceutically acceptable salt or solvate thereof, in combination with at least one pharmaceutically acceptable carrier, which includes the step of preparing the substantially optically pure omeprazole or the pharmaceutically acceptable salt or solvate thereof according to the process of any one of claims 1 to 14.

16. An omeprazole ammonium salt, wherein the ammonium moiety is derived from a chiral amine selected from the group consisting of (-)-brucine, (+)-α-methylbenzylamine, (-)-ephedrine, N,N-dimethylephedrine and bis-(1-phenylethyl)-amine.

17. An omeprazole ammonium salt having the formula (II): 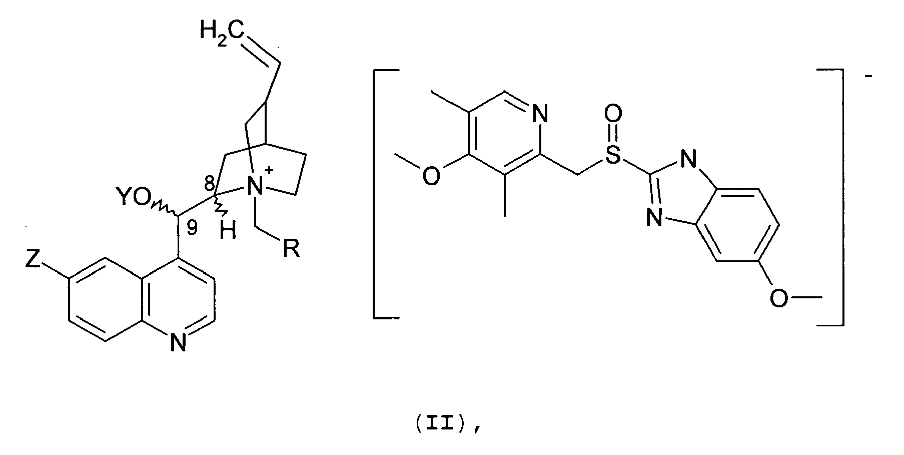 wherein:
the configuration at C(8), C(9) and the sulfur atom is independently (R) or (S);
Z is hydrogen or methoxy;
Y is benzyl or allyl; and
R is hydrogen or phenyl.

18. An omeprazole ammonium salt having the formula (III): 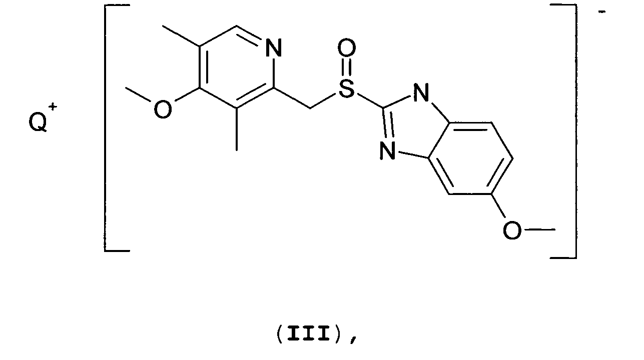 wherein the configuration at the sulfur atom is independently (R) or (S) and wherein Q⁺ is selected from the group consisting of:
